# EUROPÄISCHE PATENTSCHRIFT

(11) **EP 4 003 385 B1**
(45) Veröffentlichungstag und Bekanntmachung des Hinweises auf die Patenterteilung: **30.08.2023**
(21) Anmeldenummer: 20760775.5
(22) Anmeldetag: 31.07.2020
(51) Int. Cl.: A61K 36/25, A61K 36/53, A61K 47/00, B01D 1/00, A61K 9/08, A61K 47/40

(54) **MEMBRANFILTRATION VON PFLANZENEXTRAKTEN MITTELS CYCLODEXTRIN**
MEMBRANE FILTRATION OF PLANT EXTRACTS USING CYCLODEXTRIN
FILTRATION SUR MEMBRANE DES EXTRAITS DE PLANTES AU MOYEN DE CYCLODEXTRINE

(30) Priorität: 31.07.2019 EP 19189464
(43) Veröffentlichungstag der Anmeldung: 01.06.2022
(73) Patentinhaber: Bionorica SE, 92318 Neumarkt (DE)
(72) Erfinder: RUBNER, Moritz, 93188 Pielenhofen (DE); FERSCH, Silvia, 92339 Beilngries (DE)
(74) Vertreter: Simandi, Claus
(86) Internationale Anmeldenummer: PCT/EP2020/071738
(87) Internationale Veröffentlichungsnummer: WO 2021/019097

(56) Entgegenhaltungen:
- EP-A1- 2 621 505
- EP-B1- 2 621 505
- JP-A- H05 236 877
- N.N.: "Cefacur", , 1. Juni 2018 (2018-06-01), XP055659290, Gefunden im Internet: URL:https://www.cefak.com/fileadmin/user_u pload/pdf_ratgeber/cefacur-ratgeber.pdf [gefunden am 2020-01-20]
- WADHWA GEETIKA ET AL: "Essential oil-cyclodextrin complexes: an updated review", JOURNAL OF INCLUSION PHENOMENA AND MACROCYCLIC CHEMISTRY, KLUWER, DORDRECHT, NL, Bd. 89, Nr. 1, 21. August 2017 (2017-08-21), Seiten 39-58, XP036321530, ISSN: 1388-3127, DOI: 10.1007/S10847-017-0744-2 [gefunden am 2017-08-21] in der Anmeldung erwähnt

## Beschreibung

Die vorliegende Erfindung betrifft ein Verfahren zur Herstellung einer alkoholreduzierten bzw. alkoholfreien Zusammensetzung von mindestens einem Alkohol-enthaltenden Pflanzenextrakt mittels Membranfiltration unter Verwendung von Cyclodextrin.

Arzneipflanzen enthalten, ggfs. in bestimmten Pflanzenteilen wie Wurzeln, Blättern, Blüten oder Früchten angereichert, Inhaltsstoffe mit pharmakologischer Wirkung und bilden die Grundlage für eine beachtliche Anzahl von Arzneimitteln und Nahrungsergänzungsmitteln. Zur Gewinnung dieser Inhaltsstoffe gibt es unterschiedliche Verfahren, von denen die meisten nach dem Prinzip einer wie auch immer gearteten Extraktion einschließlich Mazeration oder Perkolation der Pflanzen mit einem geeigneten Extraktionsmittel bzw. Lösungsmittel arbeiten, zumeist jedoch Alkohol und infolge dessen eine mehr oder weniger selektive Lösung und Anreicherung bestimmter pflanzlicher Wirkstoffe oder Wirkstoffgruppen in dem Extraktionsmittel bzw. Auszug ergeben. Es werden daher Alkohol-enthaltende Pflanzenextrakte erhalten. Alkoholhaltige Arzneimittel, Auszüge und Fluidextrakte auf der Basis von Pflanzenextrakten stehen jedoch fortwährend in der Kritik. Zumeist werden für Pflanzenextrakte wässrig-ethanolische Lösungsmittel als Auszugsmittel verwendet, die 30 bis 70 % Ethanol enthalten können. Beispielsweise werden Fluidextrakte in Arzneiformen, wie Tropfen und Säfte eingebracht.

Es besteht ein hohes Bedürfnis alkoholfreie Mittel, insbesondere Arzneimittel aus Pflanzenextrakten bereitzustellen.

Die Membranfiltration ist ein druckbetriebenes Filtrationsverfahren zur selektiven Abtrennung von Molekülen mit einem Molekulargewicht von wenigstens 100 Dalton bis zu einer Größe von 5 µm i.d.R. bei niedrigen Temperaturen, z. B. Umgebungstemperatur ohne Phasenänderung.

Eine besondere Ausführungsform der Membranfiltration ist die Umkehrosmose oder Reversosmose. Die Umkehrosmose ist ein Hochdruckverfahren, das zur Abtrennung von Wasser oder Alkohol aus Prozessflüssigkeiten, insbesondere zur Konzentrierung von niedermolekularen Verbindungen eingesetzt wird. Die Umkehrosmose oder Reversosmose ist ein physikalisches Verfahren zur Konzentrierung von in Flüssigkeiten gelösten Stoffen, bei der mit Druck der natürliche Osmose-Prozess umgekehrt wird.

Weitere Ausführungsformen der Membranfiltration in Abhängigkeit der Molekülgröße und angelegten Drücken umfassen sowohl die Ultrafiltration als auch die Nano- und Mikrofiltration.

Eine im Stand der Technik bekannte lebensmitteltechnische Anwendung der Membranfiltration, insbesondere Umkehrosmose ist die Herstellung von alkoholfreiem Bier.

Im Zuge der Membranfiltration entsteht aus dem eingesetzten Fluid bzw. Feed ein Permeat (=Filtrat) aufweisend den entfernten Alkohol und Wasser, während das Retentat an der Membran zurückgehalten und folglich aufkonzentriert wird (Konzentrat).

Weiterhin beschreibt EP 2621505 B1 die Entalkoholisierung von Pflanzenextrakten, wie Thymian, Fenchel u.a. unter Einsatz von Dispergiermittel und Netzmittel, insbesondere auf der Basis von Ölen, Fetten und Fettsäuren, Lecithinen und Glyceriden.

Es besteht jedoch das Bedürfnis die Entalkoholisierung von Pflanzenextrakten weiter zu verbessern.

Cyclodextrine sind im Stand der Technik breit beschrieben, jedoch konnten die Erfinder erstmals Cyclodextrine zur Entalkoholisierung von Pflanzenextrakten vorteilhaft einsetzen. Insbesondere die Eignung von Cyclodextrinen zur Herstellung von Einschlussverbindungen erlaubt eine Vergrößerung der Molekülgröße, so dass vorteilhaft ein Rückhalten an der Membran während der Membranfiltration erreicht werden kann. Solche Moleküle können nicht abschließend vorzugsweise niedermolekulare Verbindungen sein, wie Terpene, Monoterpene, Thymol, Carvacrol, Limonen, 1,8-Cineol und 1,4-Cineol, p-Cymen, Anethol, Eugenol, Fenchon, Myristicin, Vanillin, Estragol, Zingiberol, Pinen, Linalool, Terpineol, Myrcene, Menthone, Menthol, Phellandren, Menthan, Carveol und Dihydrocarveol, Carvon, Thujon, Borneol, 3-Caren, Anethol, Estragol u.v.a..

Ferner umfasst sind die jeweiligen Stereoisomere, Diastereomere, Enantiomere, wie (+), (-), alpha, beta, R und S etc.

Daher betrifft die Erfindung ein Verfahren zur Herstellung einer alkoholreduzierten oder - freien Zusammensetzung von mindestens einem Pflanzenextrakt, wobei die Zusammensetzung einen Alkoholgehalt von weniger als 10 Vol. %, insbesondere weniger als 5 Vol. %, vorzugsweise weniger als 2,5 Vol. %, insbesondere weniger als 1,5 Vol. % oder gleich oder weniger als 5.000 ppm (0,5 Vol. %) oder gar gleich oder mehr als 0,0 % aufweist, umfassend die folgenden Schritte:
(a) Bereitstellung von mindestens einem Alkohol-enthaltenden Pflanzenextrakt,
(b) Versetzen mit mindestens einem Cyclodextrin oder Cyclodextrinderivat,
(c) Entfernen des Alkohols mittels Membranfiltration (Permeat),
(d) Entnahme der alkoholreduzierten oder alkoholfreien Zusammensetzung (Retentat).

Optional kann das erhaltene Retentat erneut in den Schritt (a) eingesetzt werden, so dass eine weitere Reduktion des Alkohols erfolgt. Das erhaltene Retentat ist ggfs. mit Wasser zu verdünnen.

Im Sinne dieser Erfindung wird unter "alkoholfrei (analkoholisch)" ein Gehalt von weniger als 1,5 Volumenprozent, vorzugsweise gleich oder weniger als 5000 ppm, gleich oder mehr als 0,0 Volumenprozent an Alkohol, insbesondere Ethanol in einer flüssigen Zusammensetzung verstanden. Ggfs. können weitere Hilfsmittel und Zusatzstoffe eingesetzt werden, wie Konservierungsmittel (u.a. Kaliumsorbat, Sorbinsäure, Natriumbenzoat), pH-Regulatoren (u.a. Citronensäure-Monohydrat), Puffer (u.a. Natriumgluconat), Lösungsvermittler (u.a. Glycerin, Monopropylenglycol, Polyethylenglycol), Viskositätserhöher (u.a. Polyvinylpyrrolidon, Xanthangummi, Natrium-Carboxymethylcellulose, Natriumalginat, Maltodextrin, Methylcellulose), Solubilisatoren (u.a. Macrogolglycerolhydroxystearat, Octenylstärkesuccinat), Viskositätserhöher (u.a. Polyvinylpyrrrolidon, Xanthangummi, Natrium-Carboyxmethylcellulose, Natriumalginat, Maltodextrin, Methylcellulose) Süßungsmittel (u.a. Saccharose, Maltitol, Sorbitol, Isomalt, Saccharin-Natrium), Aromastoffe, Lösungsmittel (gereinigtes Wasser), Farbstoffe, Antioxidantien (u.a. Ascorbinsäure), Chelatbildner (u.a. Natrium-EDTA), Filtrationshilfsstoffe (u.a. quervernetztes Polyvinylpyrrolidon), so dass ein Endprodukt alkoholfrei ist.

Im Sinne dieser Erfindung wird unter "Cyclodextrin (kurz: CD)" ein nichtreduzierendes cyclisches Saccharid verstanden, auch Cyclohexaamylose genannt, bestehend aus sechs oder mehr α-1,4-verknüpften D-Glucopyranosyleinheiten, das durch Einwirkung von Cycloglykosyltransferase (CGTase, EC 2.4.1.19) aus hydrolisierter Stärke hergestellt wird. Je nach Zahl der D-Glucopyranosyleinheiten unterscheidet man alpha, beta, gamma und delta-Cyclodextrin, welche zu Einschlußverbindungen mit einem Molekül geeignet ist. Ebenfalls erfindungsgemäß umfasst sind Cyclodextrinderivate, wie Alkylcyclodextrine oder Acylcyclodextrine, z.B. Methyl-β-cyclodextrin oder Hydroxyalkylcyclodextrine, wie z.B. 2-Hydroxypropyl-β-Cyclodextrin, wobei Hydroxygruppen einer Glucoseeinheit derivatisiert werden.

Für die Bildung von Einschlusskomplexen mit CD sind van-der-Waals-Kräfte, elektrostatische Interaktionen, Wasserstoffbrückenbindungen und Charge-Transfer-Interaktionen verantwortlich zu machen. Verdrängung von Wasser aus der Kavität und Verringerung von Konformationsspannungen spielen nur eine untergeordnete Rolle. Die Bindungsinteraktionen von α-, β- und γ-CD mit z.B. Thymol im kristallinen und im gelösten Zustand sind von Ceborska (2018) zusammengefasst. Die Interaktionen von Thymol mit α- und β-CD haben Bose et al. (2019) spektroskopisch und mit molekularen Docking- und Simulationsstudien untersucht. Demnach binden solvatisierte Thymol-Moleküle im Innern von α- und β-CD im molaren Verhältnis 1:1. Dadurch wird eine Vergrößerung der Thymol Moleküle erreicht.

Im Rahmen dieser Erfindung wird unter einem "Pflanzenextrakt" ein Vielkomponentengemisch aus Naturstoffen verstanden, welches mehr als zwei Naturstoffe, insbesondere mehr als 10 oder 100 Naturstoffe, insbesondere mehr als 200, 300, 500 oder 1.000 Naturstoffe enthält. Pflanzenextrakte können beispielsweise mittels Extraktion, Perkolation oder Mazeration aus Pflanzenmaterialien gewonnen werden. Als Extraktionsmittel können alkoholhaltige Lösungsmittel, C1-C5-Alkohole, Ethanol verwendet werden. Eine übliche Extraktion ist beispielsweise eine wässrig-ethanolische Extraktion, insbesondere ein Gemisch aus Wasser / Ethanol (50:50, 70:30, 30:70) z.B. bei 15 bis 80 Grad Celsius und Normaldruck. Der Begriff Pflanzenextrakt umfasst ebenfalls Fluidextrakte (Extractum fluidum), wobei eine flüssige Drogenzubereitung bereitgestellt wird, bei der für die Extraktion der Droge möglichst wenig Extraktionsflüssigkeit eingesetzt wird. Dies wirkt sich auf das Drogen-Extrakt-Verhältnis (DEV) aus.

Als Pflanzenmaterialien kommen die folgenden Gattungen erfindungsgemäß vorzugsweise in Betracht, solche wie Achillea, Aloe, Althaea, Angelica, Arnika, Artemisia, Cannabis, Capsicum, Carum, Caulophyllum, Centaurium, Chelidonium, Cimicifuga, Citrus, Cyclamen, Cynara, Echinacea, Equisetum, Glycyrrhiza, Guaiacum, Hedeara, Humulus, Iberis, Iris, Juglans, Lavandula, Levisticum, Lilium, Matricaria, Melissa, Mentha, Basilicum (Ocimum), Phytolacca, Pimpinella, Primula, Punica, Quercus, Rosmarinus, Rumex, Salix, Salvia, Sambucus, Silybum, Strychnos, Taraxacum, Thymus, Vaccinium, Valeriana, Vebena, Vitex, Vitis.

Als Pflanzenmaterialien kommen die folgenden Arten erfindungsgemäß vorzugsweise in Betracht, solche wie Achillea millefolium Aloe Vera, Althaea officinalis, Angelica archangelica, Arnika montana, Artemisia vulgaris, annua und absinthium, Cannabis indica, Cannabis ruderalis, Cannabis sativa, Capsicum annuum, Carum carvi, Caulophyllum thalictroides, Centaurium, Chelidonium majus, Cimicifuga racemose, Citrus reticulata, Citrus sinensis, Citrus junos, Citrus medica, Citrus maxima, Citrus aurantifolia, Citrus aurantium, Citrus hystrix, Citrus limon, Citrus paradisi, Cistus incanus, Cyclamen purpurascens, Cynara cardunculus, Echinacea purpurea und angust, Equisetum arvense, Glycyrrhiza glabra, Glycyrrhiza inflata, Glycyrrhiza uralensis, Guaiacum officinale und sanctum, Hedeara helix, Humulus lupulus, Iberis amara, , Iris, Juglans regia, Lavandula angustifolia, Levisticum officinale, Lilium tigrinum, Matricaria chamomilla, Melissa officinalis, Mentha candensis, Mentha arvensis, Mentha piperita, Ocimum basilicum, Phytolacca americana, Pimpinella anisum, Primula veris, elatior und vulgaris, Punica granatum, Quercus robur, Quercus petraea, Quercus pubescens, Rosmarinus, Rumex cripus, Rumex obtusifolius, Rumex alpinus, Rumex patientia, Rumex acetosa, Rumex acetosella, Rumex thyrsiflorus, Salix purpurea, Salix daphnoides, Salix fragilis, Salvia officinalis, Sambucus nigra, Silybum marianum, Strychnos ignatii Taraxacum officinale, Thymus vulgaris, Vaccinium macrocarpon, Vaccinium myrtillus, Valeriana officinalis, Vebena officinalis, Vitex agnus castus, Vitis vinifera.

Weiterhin ist vorteilhaft, wenn die Konzentration an Cyclodextrin im flüssigen Pflanzenextrakt oder Fluidextrakt vor Durchführung der Membranfiltration vorzugsweise 0,1-20 % m/m, vorzugsweise 3,7- 4,2 % m/m beträgt.

In einer weiteren bevorzugten Ausführungsform der Erfindung der erfindungsgemäßen Membranfiltration ist die Porengröße einer Membran ungefähr 0,0001 bis 0,001 µm. Diese Porengröße erlaubt ein Rückhalten von Molekülen an der Membran, die größer als 100-200 Dalton sind. Besonders geeignete Membranmaterialien sind nicht abschließend Polyamide, Polysulfone, Polyester, Polyethersulfon, Polyethylen, Celluloseacetat, Cellulose und Polypropylen. Eine chemische oder physikalische Modifizierung der Materialen kann die Selektivität beeinflussen.

Die erfindungsgemäße Membranfiltration, insbesondere Umkehrosmose ist ein druckbetriebenes Aufkonzentrierungsverfahren. Dabei ist die Flüssigkeit, in der die Konzentration des Alkohols gesenkt werden soll, mittels einer Membran von dem Medium getrennt, in dem die Konzentration des Alkohols erhöht werden soll. Die Membran zeichnet sich durch selektiv permeable Eigenschaften aus. Der Prozess der Osmose wird durch die Erhöhung des hydrostatischen Druckes umgedreht, wodurch ein Stoffübergang entgegen den Konzentrationsgradienten möglich ist. Durch den entstehenden Transmembrandruck wird die Membranfiltration, insbesondere Umkehrosmose angetrieben. Drücke (Druckdifferenz und/oder einem Druckgradienten und/oder hydrostatische Druckdifferenz) von 5 -80 bar, vorzugsweise 10-60 bar haben sich in Bezug auf die erfindungsgemäße Alkohol-, Ethanolreduzierung in Pflanzenextrakten mit Cyclodextrinen als besonders geeignet herausgestellt. Durch die Prozesstemperatur wird die Durchlässigkeit der Membran mitgesteuert. Als besonders geeignet erwiesen sich Temperaturen zwischen 5 - 60 °C, vorzugsweise 25-35 °C. Weiterhin ist bevorzugt, dass das Verfahren vorzugsweise kontinuierlich und in einem geschlossenen System gefahren wird, um einerseits die Prozesszeit zu minimieren und andererseits Verunreinigungen, Kreuzkontaminationen und den Verlust wichtiger Inhaltsstoffe zu verhindern.

In einer weiteren Ausführungsform der Erfindung kann eine vorteilhafte Vor- oder Nachbehandlung der Pflanzenextrakte erfolgen, solche wie Entkeimung mittels Kurzzeiterhitzung, Pasteurisierung, Ultrahocherhitzung, entkeimende Filtration, o. ä.. Ebenso ist eine Konservierung mit Konservierungsmitteln, wie z.B. Kaliumsorbat möglich.

Weiterhin betrifft die Erfindung ein Mittel, insbesondere ein Arzneimittel oder Nahrungsergänzungsmittel enthaltend einen alkoholfreien Pflanzenextrakt, wobei die Zusammensetzung einen Alkoholgehalt von weniger als 1,5 %, vorzugsweise 5.000 ppm aufweist, jedoch der Alkoholgehalt größer als Null ist, und mindestens ein Cyclodextrin oder Cyclodextrinderivat enthält, ggfs. weitere Zusatzstoffe und Hilfsmittel. Weiterhin ist bevorzugt, dass der Anteil Cyclodextrin oder Cyclodextrinderivat 0,1 - 5 Gew % beträgt (berechnet auf die Gesamtmasse oder Gesamtvolumen des Arzneimittels).

Das Arzneimittel kann vorzugsweise in flüssiger Form bereitgestellt werden, insbesondere in einer Arzneiform ausgewählt aus der Gruppe Tropfen, Saft, Sirup, Aufguss, insbesondere Rachenspray sowie Desinfektionslösungen, Nasenspray, flüssige Präparate für die Inhalation, Spüllösungen, insbesondere in Kombination mit physiologischen und hyperosmolaren Konzentrationen von Salzen oder Salzgemischen, bevorzugt Kochsalz, insbesondere Meersalz.

### Beispiele:

Die folgenden Beispiele und Figuren sollen die Erfindung näher erläutern.

### Beispiel 1:

### 1. Herstellung des Feedprodukts für die Umkehrosmose

Zunächst wurden zwei beispielhafte alkoholische Extrakte aus Thymian (Thymus) und Efeu (Hedeara) hergestellt. Zur Herstellung des Thymian-Fluidextrakts wurde als Auszugsmittel eine Mischung aus 90 %-igem Ethanol, 85 %-igem Glycerin und einer 10 %-igen Ammoniaklösung verwendet.

Für die Herstellung des Efeuextrakts wurde 70%iger Ethanol herangezogen. Durch Aufkonzentration und Vereinigung einer Teilmenge des Efeu-Auszugs wurde ein Efeu-Fluidextrakt mit 57 % (V/V) Ethanol hergestellt.

Die beiden herstellten Fluidextrakte, sowie HP-ß-Cyclodextrin wurden im Verhältnis 10: 1: 1 (Fluidextrakt Thymian : Fluidextrakt Hedera : HP-ß-Cyclodextrin) gemischt. Das vorliegende Startprodukt wies einen Ethanolgehalt von 33,091 % (V/V) Ethanol auf.

Die Mischung wurde vor Durchführung der Umkehrosmose mit Wasser im Verhältnis 1:1 verdünnt. Das so für die Umkehrosmose vorbereitete Feedprodukt enthielt 4,2 % HP-ß-Cyclodextrin und einen Ethanolgehalt von 16,304 % (V/V).

Von der vorliegenden Mischung wurde ein analytisches Chromatogramm erstellt, um das gesamte Inhaltsstoffspektrum des vorliegenden Feedprodukts festzuhalten und zu charakterisieren. Dies wird benötigt, um bei den nachfolgenden Versuchen feststellen zu können, ob sich das Inhaltsstoffspektrum des Feedprodukts verändert hat oder nicht.

### Beispiel 2:

### 2. Ethanolreduktion des Feedprodukts mit Cyclodextrin durch Membranfiltration

Zur Entalkoholisierung des Beispiel 1 beschriebenen Feedprodukts mit Cyclodextrin wurde eine für Membranfiltrationen übliche Laboranlage eingesetzt.

Zur Feststellung der Filtrationseffektivität wurde neben dem Feedprodukt das erzeugte Permeat und Retentat analytisch charakterisiert. Hierbei diente wiederum ein analytisches Chromatogramm zur Feststellung aller Inhaltsstoffe des Feedprodukts.

Ziel der Umkehrosmose war es nun, den im Feed enthaltenen Ethanol durch die Membranfiltration abzutrennen, wobei möglichst wenig an Produktinhaltsstoffen in das Permeat abfiltriert werden.

Hierzu wurden zwei Membranfiltrationsversuche mit dem gleichen Feedprodukt durchgeführt.

Im ersten Versuch wurde eine gereinigte Membranfiltrationsanlage verwendet. Beim zweiten Versuch hingegen wurde die Anlage vor dem Hauptversuch mit dem Feedprodukt vorkonditioniert. Hierbei wurde die Anlage mit eingebauter Membran vor dem Hauptversuch mit dem Feed gefahren, um die Membran mit Produkt zu benetzen. Dadurch werden alle freien Bindungsstellen der polymeren Membran mit Molekülen des Feeds gebunden. Das zur Vorkonditionierung verwendete Produkt wurde verworfen.

In der nachfolgenden Tabelle sind die erzielten Ergebnisse zusammengefasst.

**Tabelle 1: Erster Versuch mit gereinigter Membranfiltrationsanlage (ohne Vorkonditionierung):**

| | Menge [g] | Ethanolgehalt [%V/V] | Theoretischer Ethanolgehalt [%V/V] nach Mischung mit weiteren Hilfsstoffen zum Fertigarzneimittel |
|---|---|---|---|
| Vor Umkehrosmose: | | | |
| Startprodukt | 557,4 | 33,091 | 6,56 |
| Feedprodukt | 1114,7 | 16,304 | 6,52 |

| Nach Umkehrosmose: | | | |
|---|---|---|---|
| Konzentrat (Retentat) | 614,2 | 15,284 | 2,44 |
| Permeat | 474,0 | 15,550 | -- |

**Tabelle 2: Zweiter Versuch mit vorkonditionierter Membranfiltrationsanlage:**

| | Menge [g] | Ethanolgehalt [%V/V] | Theoretischer Ethanolgehalt [%V/V] nach Mischung mit weiteren Hilfsstoffen zum Fertigarzneimittel |
|---|---|---|---|
| Vor Umkehrosmose: | | | |
| Startprodukt | 559,9 | 33,091 | 6,56 |
| Feedprodukt | 1119,8 | 16,236 | 6,49 |

| Nach Umkehrosmose: | | | |
|---|---|---|---|
| Konzentrat (Retentat) | 794,1 | 16,087 | 2,13 |
| Permeat | 298,7 | 15,502 | --- |

Die erzeugten Konzentrate, die optimalerweise die gesamten Produktinhaltsstoffe enthalten, stellen das Produkt dar. Das gewonnene Permeat, welches im Idealfall nur Ethanol und Wasser und keine Produktinhaltsstoffe enthält, wird hingegen als Abfallprodukt verworfen. Das Ausgangsvolumen wurde durch die Abfiltration des Permeats reduziert.

Somit konnten bei den beiden durchgeführten Versuchen Konzentrate gewonnen werden, welche nun nur noch ca. 15-16 % (V/V) Ethanol enthalten, der Alkoholgehalt konnte somit von 33,091 % (V/V) auf 15-16 % (V/V) reduziert werden.

Durch die Vermischung mit weiteren Hilfs- und Zusatzstoffen, wie Konservierungsmittel (u.a. Kaliumsorbat, Sorbinsäure, Natriumbenzoat), pH-Regulatoren (u.a. Citronensäure-Monohydrat), Puffer (u.a. Natriumgluconat), Lösungsvermittler (u.a. Glycerin, Monopropylenglycol, Polyethylenglycol), Viskositätserhöher (u.a. Polyvinylpyrrolidon, Xanthangummi, Natrium-Carboxymethylcellulose, Natriumalginat, Maltodextrin, Methylcellulose), Solubilisatoren (u.a. Macrogolglycerolhydroxystearat, Octenylstärkesuccinat), Viskositätserhöher (u.a. Polyvinylpyrrrolidon, Xanthangummi, Natrium-Carboyxmethylcellulose, Natriumalginat, Maltodextrin, Methylcellulose) Süßungsmittel (u.a. Saccharose, Maltitol, Sorbitol, Isomalt, Saccharin-Natrium), Aromastoffe, Lösungsmittel (gereinigtes Wasser), Farbstoffe, Antioxidantien (u.a. Ascorbinsäure), Chelatbildner (u.a. Natrium-EDTA), Filtrationshilfsstoffe (u.a. quervernetztes Polyvinylpyrrolidon) zu einem Arzneimittelsaft (z.B. für Kinder) kann statt den erreichten 5-6 % (V/V) Ethanol nur noch ca. 2-3 % (V/V) Ethanol enthalten sein.

Zur weiteren Reduktion des Ethanols wird das Konzentrat kontinuierlich mit Wasser verdünnt und weiter dem erfindungsgemäßen Umkehrosmoseverfahren unterzogen. Die EthanolReduktion wird bis zu einem Restwert von ca. 2 % Ethanol durchgeführt. Durch anschließende Vermischung mit den vorgenannten Hilfs- und Zusatzstoffen zum Fertigarzneimittel kann eine alkoholfreie Fertigarzneimittelrezeptur mit weniger als 5000 ppm Ethanolgehalt erhalten werden.

Die Auswertung der analytischen Chromatogramme der Proben aus den Versuchen hat ergeben, dass die Inhaltsstoffprofile von Feed und Konzentrat im Vergleich zum Startprodukt unverändert bleibt. Im Permeat konnten keinerlei Substanzen nachgewiesen werden. Qualitativ ist das erzeugte Konzentrat identisch mit dem Feed und somit mit dem eingesetzten Startprodukt.

Die Umkehrosmose einer Extraktmischung unter Zusatz von Cyclodextrin ist somit äußerst effektiv und vorteilhaft bezüglich des Rückhalts an Produktinhaltsstoffen im Retentat.

### Beispiel 3:

### 3. Untersuchung des Einflusses von Cyclodextrin bei der Membranfiltration

In einem weiteren Versuch wurde gezielt der Einfluss des Cyclodextrins im Feedprodukt bei Durchführung einer Membranfiltration untersucht. Ganz entscheidend ist hierbei, ob durch den Zusatz von Cyclodextrin das im Feedprodukt enthaltene Thymol quantitativ stärker im Retentat zurückgehalten werden kann und somit in geringerem Maße in das Permeat abfiltriert wird. Thymol stellt einen sehr wichtigen, pharmakologisch relevanten Inhaltsstoff von Thymian dar, gehört jedoch zu den niedermolekularen Verbindungen, sodass es besonders leicht in das Permeat abfiltriert wird.

Vor Versuchsbeginn wurde als Startprodukt wiederum eine Mischung aus Fluidextrakt Thymian, Fluidextrakt Efeu und HP-β-Cyclodextrin hergestellt (Mischung im Verhältnis 10:1:1, wie im Kapitel 1 beschrieben).

Um den Einfluss des Cyclodextrins auf die Membranfiltration gezielt untersuchen zu können, wurde die gleiche Fluidextraktmischung hergestellt, jedoch ohne Zusatz von HP-ß-Cyclodextrin (Mischung Fluidextrakt Thymian mit Fluidextrakt Efeu im Verhältnis 10 :1).

Beide hergestellten Startprodukte wurden 1:1 mit Wasser verdünnt, sodass zwei identische Feedprodukte einmal mit und einmal ohne Cyclodextrin hergestellt wurden.

Das so vorbereitete Feedprodukt mit Cyclodextrin enthielt 3,75 % HP-β-Cyclodextrin und hatte einen Ethanolgehalt von 14,631 % (V/V).

Das Feedprodukt ohne Cyclodextrin wies einen gemessenen Ethanolgehalt von 14,073 % (V/V) auf.

Für die Durchführung der Versuche kam wiederum eine für Membranfiltrationen übliche Laboranlage zu Einsatz, mit welcher im gleichen Versuchsbedingungen verschiedene Membrane gleichzeitig getestet werden können. Die Anlage wurde für den Versuch mit vier verschiedenen Umkehrosmosemembranen (Membran A-D) bestückt.

Im ersten Durchlauf wurde die Membranfiltration mit dem Cyclodextrin-versetzten Feedprodukt durchgeführt. Für den zweiten Durchlauf wurden neue Membrane A-D eingesetzt mit den gleichen Bedingungen das Feedprodukt ohne Cyclodextrin filtriert.

Für eine sensitive, analytische Versuchsbegleitung wurde von allen erzeugten Permeaten eine quantitative Thymolgehaltsbestimmung durchgeführt.

**Tabelle 3: In der folgenden Tabelle sind die Ergebnisse der Versuchsreihe zusammengefasst:**

| Bezeichnung | | Feed mit/ohne HP-β-Cyclodextrin | Thymolgehalt Feed [mg/100 mg] | Thymolgehalt Permeat [mg/100 mg] | Wiederfindung Thymol [%] |
|---|---|---|---|---|---|
| Permeat | Membran A | Mit | 0,01927 | 0,00001 | 0,05 |
| Permeat | Membran A | Ohne | 0,02261 | 0,00031 | 1,37 |
| Permeat | Membran B | Mit | 0,01927 | 0,00001 | 0,05 |
| Permeat | Membran B | Ohne | 0,02261 | 0,00035 | 1,55 |
| Permeat | Membran C | Mit | 0,01927 | 0,00052 | 2,70 |
| Permeat | Membran C | Ohne | 0,02261 | 0,00352 | 15,57 |
| Permeat | Membran D | Mit | 0,01927 | 0,00094 | 4,88 |
| Permeat | Membran D | Ohne | 0,02261 | 0,00477 | 21,10 |

Die Auswertung der analytischen Ergebnisse erfolgte anhand der berechneten Wiederfindungen an Thymol im Permeat. D.h. je geringer die Wiederfindung im Permeat ist, desto geringer ist der Übergang von Thymol in das Permeat und desto höher der Rückhalt an Thymol auf der Retentatseite.

Die Wiederfindungen des Thymols im Permeat waren bei Einsatz von Feed mit Cyclodextrin deutlich geringer als bei den Versuchen ohne Cyclodextrin. Somit ist der Übergang an Thymol in das Permeat mit Einsatz von Cyclodextrin besonders gering.

Dieses Ergebnis konnte durchgängig mit allen Membranen erzielt werden, d.h. der Effekt, dass Cyclodextrin das Thymol auf der Retentatseite verstärkt zurückhält und somit im Permeat weniger Thymol zu finden ist, konnte unabhängig von der eingesetzten Membran festgestellt werden. Verstärkt wird diese Erkenntnis, da die Versuchsreihe mit dem gleichen Ausgangsprodukt, unter gleichen Versuchsbedingungen und mit unterschiedlichen Membranen durchgeführt wurde. D.h. systembedingte Schwankungen können ausgeschlossen werden.

### Beispiel 4:

### 4. Durchführung einer Entalkoholisierung

Ziel eines weiteren Versuchs ist es, eine mit Cyclodextrin versetzte Extraktmischung (Startprodukt) zu Entalkoholisieren, wobei der Ethanolgehalt des Startprodukts von 25 % (m/m) auf mindestens ≤ 3 % (m/m) reduziert werden. Folglich kann aus diesem entalkoholisierten Konzentrat durch Mischung mit weiteren Hilfsstoffen ein Fertigarzneimittel mit ≤ 0,6 % (V/V) bzw. ≤ 0,5 % (m/m) Ethanol hergestellt werden.

Obwohl in diesem Versuch das Startprodukt einer stärkeren Entalkoholisierung unterzogen wird, soll das Inhaltsstoffspektrum von Startprodukt zum Konzentrat analytisch vergleichbar bzw. identisch bleiben.

Der vorliegende Versuch wurde mit einer größeren Produktmenge und mit einer für Membranfiltrationen üblichen Technikum-Umkehrosmoseanlage mit Membranmodul durchgeführt.

Für den Versuch wurden zwei alkoholische Extrakte aus Thymian und Efeu verwendet, welche auf die gleiche Art und Weise hergestellt wurden wie es in Beispiel 1 beschrieben ist. Die beiden Fluidextrakte wurden im Verhältnis im Verhältnis 10:1:1 mit HP-ß-Cyclodextrin vermischt. Im vorliegenden Startprodukt konnte ein Ethanolgehalt von 32,79 % (V/V) analysiert werden. Außerdem wurde vom Startprodukt ein analytisches Chromatogramm erstellt, um das gesamte Inhaltsstoffspektrum und somit die ursprüngliche Qualität festzuhalten.

### Vorkonditionierung:

Vor dem Entalkoholisierungsprozess wurde eine Vorkonditionierung der Umkehrosmoseanlage durchgeführt. Hierzu wurde die Anlage mit eingebautem Membranmodul mit einer Startprodukt-Wasser-Mischung (60,6 % Startprodukt, 39,4 % Wasser) im Kreislauf gefahren, um die polymere Struktur des Membranmoduls mit Produkt zu benetzen. Die zur Vorkonditionierung verwendete Startprodukt-Wasser-Mischung wurde verworfen.

### Entalkoholisierung:

Nach der durchgeführten Vorkonditionierung wurde der eigentliche Entakoholisierungsprozess begonnen. Für die Entalkoholisierung wurde ein Feedmischung hergestellt aus 19,35 % Startprodukt und 80,65 % Wasser. Das vorliegende Feedprodukt wies einen Ethanolgehalt von 6,31 % (V/V) Ethanol auf und enthielt 1,6 % HP-β-Cyclodextrin.

Während dem Umkehrosmoseprozess wurde dem Konzentrat kontinuierlich Wasser zugeführt, um kontinuierlich eine Reduktion des Ethanolgehalts im Konzentrat zu bewirken. Die Umkehrosmose wurde so lange durchgeführt bis das Konzentrat einen Ethanolgehalt von ≤ 3 % (m/m) aufwies und die Konzentratmenge der Startproduktmenge entsprach.

In der nachfolgenden Tabelle 4 sind die Ergebnisse des Versuchs zusammengefasst:

| | Menge [kg] | Ethanolgehalt | Ethanolgehalt nach Mischung mit weiteren Hilfsstoffen zum Fertigarzneimittel |
|---|---|---|---|
| Vor Umkehrosmose: | | | |
| Startprodukt | 11,868 | 32,79 % (V/V) // 25,3 % (m/m) | 6,12 % (V/V) // 4,3 % (m/m) |
| Feedprodukt | 61,333 | 6,31 % (V/V) // 5,0 % (m/m) | --- |

| Nach Umkehrosmose: | | | |
|---|---|---|---|
| Konzentrat (Retentat) | 11,162 | 3,81 % (V/V) // 2,8 % (m/m) | 0,62 % (V/V) // 0,4 % (m/m) |
| Permeat | 103,117 | 4,68 % (V/V) // 3,7 % (m/m) | --- |

Die vorliegenden Ergebnisse zeigen, dass die Entalkoholisierung des Startprodukts erfolgreich war und ein Ethanolgehalt von ≤ 3 % (m/m) im Konzentrat erzielt werden konnte. Das aus dem Konzentrat hergestellte Fertigarzneimittel wies einen Ethanolgehalt von 0,4 % (m/m) Ethanol auf.

Im Bereich der pflanzlichen Wirkstoffe bringt die ethanolisch-wässrige Extraktion ein deutlich größeres Inhaltsstoffspektrum in Lösung als bei einer rein wässrigen Extraktion. Diese Bandbreite an Wirk- und Inhaltsstoffen gilt es durch schonende, anschließende Entalkoholisierung zu bewahren und so die Elutionskraft des Ethanols zu konservieren. Die analytischen Untersuchungen der Proben aus den Versuchen fokussierten daher die Prüfung der Änderungen der Gesamtzusammensetzung der komplexen Mischungen durch die Umkehrosmose in Verbindung mit Cyclodextrin.

Als Zusammenfassung der Erkenntnisse des vorliegenden Modellversuchs mit Thymian und Efeu kann auch in diesem Versuch wieder bestätigt werden, dass die Entalkoholisierung des Startprodukts (Ausgangsmischung bestehend aus Thymian Fluidextrakt, Efeublätter Fluidextrakt und Cyclodextrin) erfolgreich war. Der initiale Ethanol-Gehalt von 25 % (m/m) konnte auf ca. 2,8 % (m/m) reduziert werden.

Die anderen Komponenten im Startprodukt sind am Ende des Prozesses qualitativ und quantitativ praktisch unverändert.

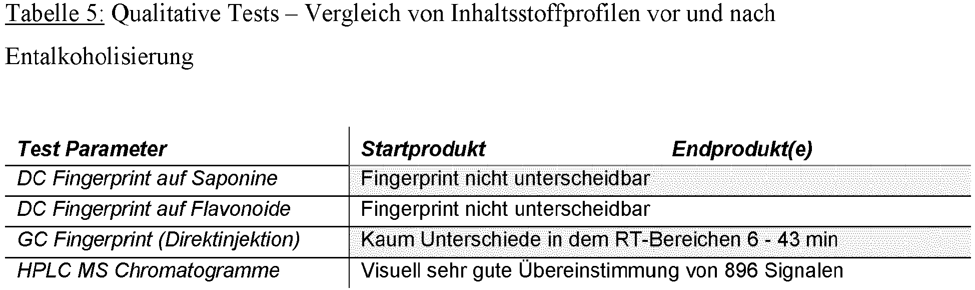

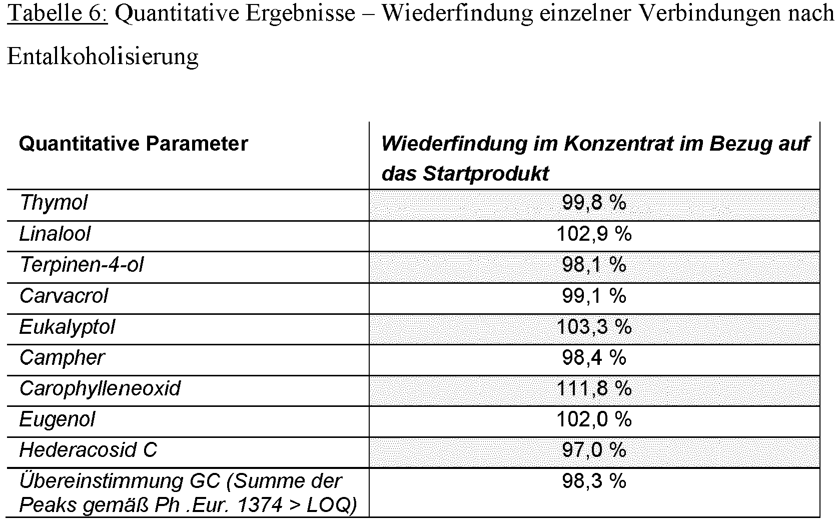

### Fingerprints:

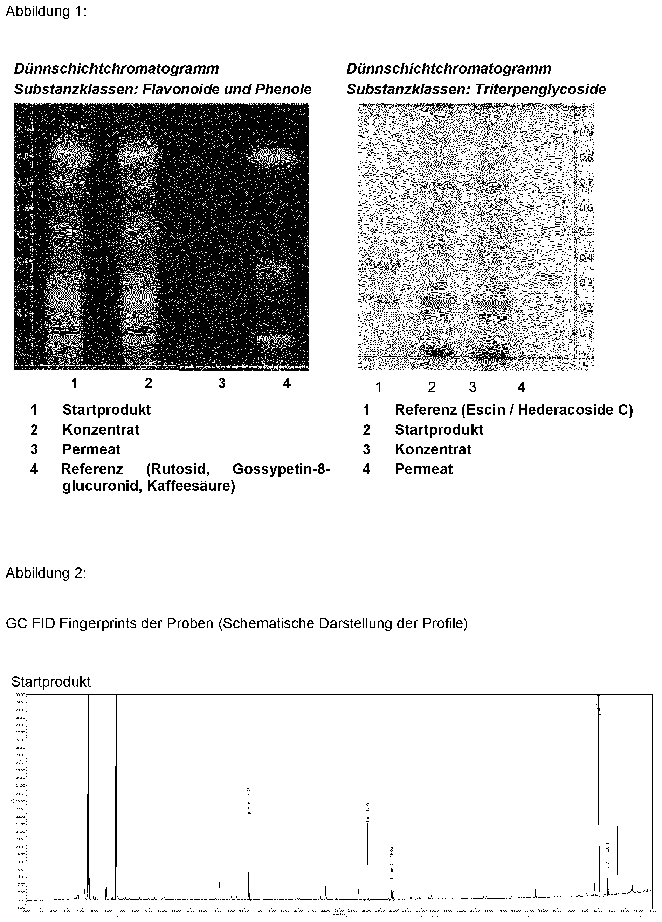

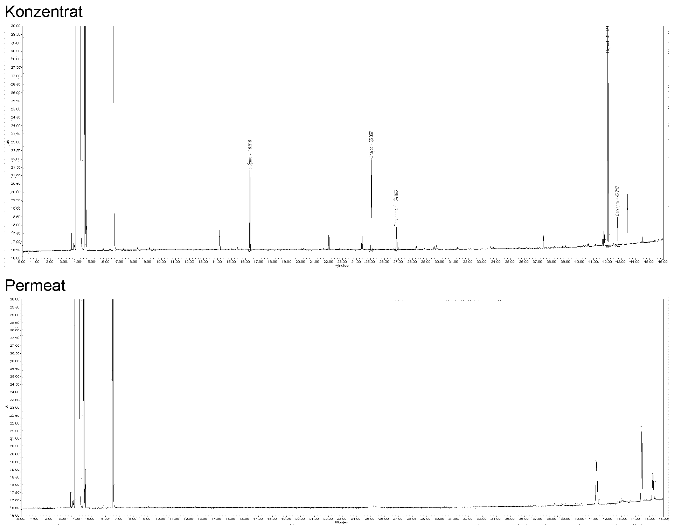

Neben den quantitativen Methoden wurden auch qualitative Fingerprint Methoden zum Profilvergleich herangezogen. Gerade bei der Substanzklasse der Flavonoide sowie Saponine zeigt sich die Effizienz der Technik. Die auch für diese Substanzklassen beschriebene Cyclodextrin vermittelte Komplexierung verhindert einen Verlust dieser Substanzen bei der Entalkoholisierung auf die Membran-Gegenseite ins Permeat (siehe Abb. 1).

### Abb. 1 zeigt Dünnschichtchromatogramme (DC-Fingerprint).

Die Methode mit Direkt-Injektion, angelehnt an die Ph. Eur. Prüfung auf Ätherische Öle des Thymians wird gewöhnlich im Retentionszeit-Bereich 6 - 43 min ausgewertet. Die präsentierten Chromatogramme in Abb. 2 zeigen den Vergleich der Gesamt-Chromatogramme für die Proben Startprodukt, Konzentrat und Permeat.

In dem oben definierten Auswertebereich, in welchem die volatilen Bestandteile der Proben erfasst werden (u.a. äth. Öl Komponenten), sind die Chromatogramme vom Startprodukt und Konzentrat praktisch identisch. Im Permeat sind in diesem Bereich keine Signale erkennbar.

### Abb. 2 zeigt GC FID Fingerprints.

### Literatur:

- Bose, A., Sengupta, P., Pal, U., Senapati, S., Ahsan, M., Roy, S., Das, U., Sen, K.: Encapsulation of thymol in cyclodextrin nano-cavities: a multi spectroscopic and theoretical study. Spectrochim. Acta Part A: Mol. Biomol. Spectr. 2019, 208, 339-348.
- Brewster, M.E., Loftsson, T.: Cyclodextrins as pharmaceutical solubilizers. Adv. Drug Deliv. Rev. 2007, 59, 645-666.
- Ceborska, M.: Structural investigation of solid state host/guest complexes of native cyclodextrins with monoterpenes and their simple derivatives. J. Mol. Struct. 2018, 1165, 62-70.
- Davis, M.E., Brewster, M.E.: Cyclodextrin-based pharmaceutics: past, present and future. Nature Reviews 2004, 3, 1023-1035.
- de Oliveira-Filho, e Silva, A.R.A., de Azevedo Moreira, R., Nogueira, N.A.P.: Biological activities and pharmacological applications of cyclodextrins complexed with essential oils and their volatile components: a systematic review. Curr. Pharm. Design 2018, 24 (33), 3951-3963.
- Lima, P.S.S., Lucchese, A.M., Araújo-Filho, H.G., Menezes, P.P., Araujo, A.A.S., Quintans-Junior, L.J., Quintans, J.S.S.: Inclusion of terpenes in cyclodextrins: preparation, characterization and pharmacological approaches. Carbohydr. Polym. 2016, 151, 965-987.
- Loftsson, T., Duchene, D.: Cyclodextrins and their pharmaceutical applications. Int. J. Pharmaceutics 2007, 329, 1-11.
- Marques, H.M.C.: A review on cyclodextrin encapsulation of essential oils and volatiles. Flavour Fragr. J. 2010, 25, 313-326.
- Pivetta, T.P., Simoes, S., Araujo, MM., Carvalho, T., Arruda, C., Marcato, P.D.: Development of nanoparticles from natural lipids for topical delivery of thymol: investigation of its anti-inflammatory properties. Colloids Surf. B Biointerfaces 2018, 164, 281-290.
- Szejtli, J.: Medicinal applications of cyclodextrins. Med. Res. Rev. 1994, 14 (3), 353-386.
- Wadhwa, G., Kumar, S., Chhabra, L., Mahant, S., Rao, R.: Essential oil-cyclodextrin complexes: An updated review. J. Incl. Phenom. Macrocycl. Chem. 2017, 89, 39-58.

## Patentansprüche

1. Verfahren zur Herstellung einer Zusammensetzung von mindestens einem Pflanzenextrakt, wobei die Zusammensetzung einen Alkoholgehalt von weniger als 10 Vol. %, insbesondere 5 Vol. % aufweist,
umfassend die folgenden Schritte:
(a) Bereitstellung von mindestens einem Alkohol-enthaltenden Pflanzenextrakt,
(b) Versetzen mit mindestens einem Cyclodextrin oder Cyclodextrinderivat,
(c) Entfernen des Alkohols mittels Membranfiltration (Permeat),
(d) Entnahme der alkoholreduzierten Zusammensetzung (Retentat).

2. Verfahren zur Herstellung einer Zusammensetzung von mindestens einem Pflanzenextrakt nach Anspruch 1, wobei die Zusammensetzung einen Alkoholgehalt von weniger als 1,5 Vol. %, insbesondere 5.000 ppm aufweist, wobei in Schritt (d) eine alkoholfreie Zusammensetzung entnommen wird.

3. Verfahren zur Herstellung einer Zusammensetzung von mindestens einem Pflanzenextrakt nach Anspruch 1 oder Anspruch 2, wobei das Retentat aus Schritt (d) in Schritt (a) eingesetzt wird, und ggfs. mit Wasser verdünnt wird.

4. Verfahren zur Herstellung einer Zusammensetzung von mindestens einem Pflanzenextrakt nach einem der Ansprüche 1 bis 3, wobei die Membranfiltration in Schritt (c) eine Ultrafiltration, Nanofiltration oder Umkehrosmose ist.

5. Verfahren zur Herstellung einer Zusammensetzung von mindestens einem Pflanzenextrakt nach einem der Ansprüche 1 bis 4, wobei das Cyclodextrin oder Cyclodextrinderivat in Schritt (b) ausgewählt ist aus der Gruppe alpha, beta, gamma und delta-Cyclodextrin, Alkylcyclodextrine, Acylcyclodextrine, Methyl-β-cyclodextrin, Hydroxyalkylcyclodextrine, 2-Hydroxypropyl-β-Cyclodextrin.

6. Verfahren zur Herstellung einer Zusammensetzung von mindestens einem Pflanzenextrakt nach einem der Ansprüche 1 bis 5, wobei die Konzentration an Cyclodextrin im flüssigen Pflanzenextrakt oder Fluidextrakt vor Durchführung der Membranfiltration 0,1 - 20 % m/m, insbesondere 3,7- 4,2 % m/m beträgt.

7. Verfahren zur Herstellung einer Zusammensetzung von mindestens einem Pflanzenextrakt nach einem der Ansprüche 1 bis 6, wobei die Membranfiltration, insbesondere Umkehrosmose mit einer Druckdifferenz und/oder einem Druckgradienten und/oder hydrostatische Druckdifferenz im Bereich von 5 - 80 bar durchgeführt wird.

8. Verfahren zur Herstellung einer Zusammensetzung von mindestens einem Pflanzenextrakt nach einem der Ansprüche 1 bis 7, wobei die Membranfiltration, insbesondere Umkehrosmose mit einer Prozesstemperatur zwischen 5 - 60 °C durchgeführt wird.

9. Verfahren zur Herstellung einer Zusammensetzung von mindestens einem Pflanzenextrakt nach einem der Ansprüche 1 bis 8, wobei der mindestens eine Pflanzenextrakt aus einer Gattung ausgewählt ist aus der Gruppe Achillea, Aloe, Althaea, Angelica, Arnika, Artemisia, Cannabis, Capsicum, Carum, Caulophyllum, Centaurium, Chelidonium, Cimicifuga, Citrus, Cyclamen, Cynara, Echinacea, Equisetum, Glycyrrhiza, Guaiacum, Hedeara, Humulus, Iberis, Iris, Juglans, Lavandula, Levisticum, Lilium, Matricaria, Melissa, Mentha, Basilicum (Ocimum), Phytolacca, Pimpinella, Primula, Punica, Quercus, Rosmarinus, Rumex, Salix, Salvia, Sambucus, Silybum, Strychnos, Taraxacum, Thymus, Vaccinium, Valeriana, Vebena, Vitex, Vitis.

10. Verfahren zur Herstellung einer Zusammensetzung von mindestens einem Pflanzenextrakt nach einem der Ansprüche 1 bis 8, wobei der mindestens eine Pflanzenextrakt aus einer Gattung ausgewählt ist aus der Gruppe Thymus und Hedera.

11. Arzneimittel oder Nahrungsergänzungsmittel enthaltend einen alkoholfreien Pflanzenextrakt, wobei die Zusammensetzung einen Ethanolgehalt von weniger als 1,5 Vol. %, vorzugsweise 5.000 ppm aufweist, jedoch der Ethanolgehalt größer als 0,0 Vol. % ist, und mindestens ein Cyclodextrin oder Cyclodextrinderivat enthält, ggfs. weitere Zusatzstoffe und Hilfsmittel.

12. Arzneimittel oder Nahrungsergänzungsmittel nach Anspruch 11 erhältlich nach einem Verfahren gemäß den Ansprüchen 1 bis 10.

13. Arzneimittel oder Nahrungsergänzungsmittel enthaltend einen alkoholfreien Pflanzenextrakt nach Anspruch 11 oder Anspruch 12, wobei der Anteil an Cyclodextrin oder Cyclodextrinderivat 0,1 - 5 Gew. % beträgt.

14. Arzneimittel oder Nahrungsergänzungsmittel enthaltend einen alkoholfreien Pflanzenextrakt nach einem der Ansprüche 11 bis 13 in flüssiger Form, insbesondere in einer Arzneiform ausgewählt aus der Gruppe Tropfen, Saft, Sirup, Aufguss, Rachenspray sowie Desinfektionslösungen, Nasenspray, flüssige Präparate für die Inhalation, Spüllösungen, insbesondere in Kombination mit physiologischen und hyperosmolaren Konzentrationen von Salzen oder Salzgemischen, bevorzugt Kochsalz, insbesondere Meersalz.

## Claims

1. A method for producing a composition of at least one plant extract, the composition having an alcohol content of less than 10%, in particular 5%, comprising the following steps:
(a) providing at least one alcohol-containing plant extract,
(b) adding at least one cyclodextrin or cyclodextrin derivative,
(c) removing the alcohol by means of membrane filtration (permeate),
(d) removing the alcohol-reduced composition (retentate).

2. The method for producing a composition of at least one plant extract according to claim 1, wherein the composition has an alcohol content of less than 1.5%, in particular 5,000 ppm, wherein an alcohol-free composition is removed in step (d).

3. The method for producing a composition of at least one plant extract according to claim 1 or claim 2, wherein the retentate from step (d) is used in step (a) and, is optionally diluted with water.

4. The method for producing a composition of at least one plant extract according to any one of claims 1 to 3, wherein the membrane filtration in step (c) is an ultrafiltration, nanofiltration or reverse osmosis.

5. The method for producing a composition of at least one plant extract according to any one of claims 1 to 4, wherein the cyclodextrin or cyclodextrin derivative in step (b) is selected from the group alpha, beta, gamma and delta-cyclodextrin, alkylcyclodextrins, acylcyclodextrins, methyl-β-cyclodextrin, hydroxyalkyl cyclodextrins, 2-hydroxypropyl-β-cyclodextrin.

6. The method for producing a composition of at least one plant extract according to any one of claims 1 to 5, wherein the concentration of cyclodextrin in the liquid plant extract or fluid extract before the membrane filtration is carried out is 0.1-20% m/m, in particular 3.7-4.2% m/m.

7. The method for producing a composition of at least one plant extract according to any one of claims 1 to 6, wherein the membrane filtration, in particular reverse osmosis, is carried out with a pressure difference and/or a pressure gradient and/or hydrostatic pressure difference in the range of 5-80 bar.

8. The method for producing a composition of at least one plant extract according to any one of claims 1 to 7, wherein the membrane filtration, in particular reverse osmosis, is carried out at a process temperature between 5 - 60°C.

9. The method for producing a composition of at least one plant extract according to any one of claims 1 to 8, wherein the at least one plant extract is selected from a genus from the group Achillea, Aloe, Althaea, Angelica, Arnika, Artemisia, Cannabis, Capsicum, Carum, Caulophyllum, Centaurium, Chelidonium, Cimicifuga, Citrus, Cyclamen, Cynara, Echinacea, Equisetum, Glycyrrhiza, Guaiacum, Hedeara, Humulus, Iberis, Iris, Juglans, Lavandula, Levisticum, Lilium, Matricaria, Melissa, Mentha, Basilicum (Ocimum), Phytolacca, Pimpinella, Primula, Punica, Quercus, Rosmarinus, Rumex, Salix, Salvia, Sambucus, Silybum, Strychnos, Taraxacum, Thymus, Vaccinium, Valeriana, Vebena, Vitex, Vitis.

10. The method for producing a composition of at least one plant extract according to any one of claims 1 to 8, wherein the at least one plant extract is selected from a genus from the group Thymus and Hedeara.

11. A medicine or food supplement containing an alcohol-free plant extract, the composition having an alcohol content of less than 1.5%, preferably 5,000 ppm, but the alcohol content being greater than zero, and containing at least one cyclodextrin or cyclodextrin derivative, optionally further additives and auxiliaries.

12. The medicines or food supplement according to claim 11 obtainable by a method according to claims 1 to 10.

13. The medicine or food supplement containing an alcohol-free plant extract according to claim 11 or claim 12, wherein the proportion of cyclodextrin or cyclodextrin derivative is 0.1 - 5% by weight.

14. The medicine or food supplement containing an alcohol-free plant extract according to any one of claims 11 to 13 in liquid form, in particular in a medicinal form selected from the group of drops, juice, syrup, infusion, throat spray and disinfectant solutions, nasal sprays, liquid preparations for inhalation, rinsing solutions, in particular in combination with physiological and hyperosmolar concentrations of salts or salt mixtures, preferably table salt, in particular sea salt.

## Revendications

1. Procédé de fabrication d'une composition d'au moins un extrait de plante, dans lequel la composition présente une teneur en alcool inférieure à 10 % en volume, notamment 5 % en volume,
comprenant les étapes suivantes :
(a) préparation d'au moins un extrait de plante contenant de l'alcool,
(b) réaction avec au moins une cyclodextrine ou un dérivé de cyclodextrine,
(c) retrait de l'alcool au moyen d'une filtration sur membrane (perméat),
(d) récupération de la composition avec l'alcool retiré (rétentat).

2. Procédé de fabrication d'une composition d'au moins un extrait de plante selon la revendication 1, dans lequel la composition présente une teneur en alcool inférieure à 1,5 % en volume, notamment 5000 ppm, dans lequel on prélève une composition exempte d'alcool dans l'étape (d).

3. Procédé de fabrication d'une composition d'au moins un extrait de plante selon la revendication 1 ou la revendication 2, dans lequel le rétentat provenant de l'étape (d) est employé dans l'étape (a), et est éventuellement dilué avec de l'eau.

4. Procédé de fabrication d'une composition d'au moins un extrait de plante selon l'une des revendications 1 à 3, dans lequel la filtration sur membrane dans l'étape (c) est une ultrafiltration, une nanofiltration ou une osmose inverse.

5. Procédé de fabrication d'une composition d'au moins un extrait de plante selon l'une des revendications 1 à 4, dans lequel la cyclodextrine ou le dérivé de cyclodextrine dans l'étape (b) est choisi dans le groupe des alpha-, bêta-, gamma- et delta-cyclodextrines, des alkyl cyclodextrines, des acyl cyclodextrines, de la méthyl β-cyclodextrine, des hydroxy alkyle cyclodextrines, de la 2-hydroxy propyl β-cyclodextrine.

6. Procédé de fabrication d'une composition d'au moins un extrait de plante selon l'une des revendications 1 à 5, dans lequel la concentration en cyclodextrine dans l'extrait de plante liquide ou l'extrait de fluide avant l'exécution de la filtration sur membrane s'élève de 0,1 à 20 % m/m, notamment 3,7 à 4,2 % m/m.

7. Procédé de fabrication d'une composition d'au moins un extrait de plante selon l'une des revendications 1 à 6, dans lequel la filtration sur membrane, notamment l'osmose inverse, est exécutée avec une différence de pression, et/ou un gradient de pression, et/ou une différence de pression hydrostatique, dans la plage de 5 à 80 bar.

8. Procédé de fabrication d'une composition d'au moins un extrait de plante selon l'une des revendications 1 à 7, dans lequel la filtration sur membrane, notamment l'osmose inverse, est exécutée avec une température de processus entre 5 et 60 °C.

9. Procédé de fabrication d'une composition d'au moins un extrait de plante selon l'une des revendications 1 à 8, dans lequel l'au moins un extrait de plante est choisi un genre choisi dans le groupe des *Achillea, Aloe, Althaea, Angelica, Arnica, Artemisia, Cannabis, Capsicum, Carum, Caulophyllum, Centaurium, Chelidonium, Cimifuga, Citrus, Cyclamen, Cynara, Echinacea, Equisetum, Glycyrrhiza, Guaiacum, Hedeara, Humulus, Ibéris, Iris, Juglans, Lavandula, Levisticum, Lilium, Matricaria, Melissa, Mentha, Basilicum (Ocimum), Phytolacca, Pimpinella, Primula, Punica, Quercus, Rosmarinus, Rumex, Salix, Salvia, Sambucus, Silybum, Strychnos, Taraxacum, Thymus, Vaccinium, Valeriana, Vebena, Vitex, Vitis..*

10. Procédé de fabrication d'une composition d'au moins un extrait de plante selon l'une des revendications 1 à 8, dans lequel l'au moins un extrait de plante est choisi dans un genre du groupe du thym et du lierre.

11. Produit pharmaceutique ou complément alimentaire contenant un extrait de plante exempt d'alcool, dans lequel la composition présente une teneur en éthanol inférieure à 1,5 % en volume, de préférence 5000 ppm, bien que la teneur en éthanol soit supérieure à 0,0 % en volume, et contient au moins une cyclodextrine ou un dérivé de cyclodextrine, éventuellement d'autres additifs ou produits auxiliaires.

12. Produit pharmaceutique ou complément alimentaire selon la revendication 11 pouvant être obtenu d'après un procédé selon les revendications 1 à 10.

13. Produit pharmaceutique ou complément alimentaire contenant un extrait de plante exempt d'alcool selon la revendication 11 ou la revendication 12, dans lequel la teneur en cyclodextrine ou en dérivé de cyclodextrine est de 0,1 à 5 % en poids.

14. Produit pharmaceutique ou complément alimentaire contenant un extrait de plante exempt d'alcool selon l'une des revendications 11 à 13, sous forme liquide ; notamment sous une forme pharmaceutique choisie dans le groupe des gouttes, des jus, des sirops, des infusions, des spray pour la gorge ainsi que des solutions désinfectantes, du spray nasal, des préparations liquides pour l'inhalation, des solutions de lavage, notamment en combinaison avec des concentrations physiologiques et hyperosmolaire de sels ou de mélanges de sels, de préférence de sel de cuisine, notamment de sel marin.
